(19) Europäisches Patentamt
European Patent Office
Office européen des brevets

(11) Numéro de publication : **0 563 025 A1**

# (12) DEMANDE DE BREVET EUROPEEN

(21) Numéro de dépôt : **93870054.9**

(51) Int. Cl.[5] : **B01J 23/84**

(22) Date de dépôt : **24.03.93**

(30) Priorité : **25.03.92 BE 9200290**

(43) Date de publication de la demande :
**29.09.93 Bulletin 93/39**

(84) Etats contractants désignés :
**BE CH DE ES FR GB IT LI NL**

(71) Demandeur : **UNIVERSITE CATHOLIQUE DE LOUVAIN**
**Place de l'Université, 1**
**B-1348 Louvain la Neuve (BE)**

(72) Inventeur : **Blangenois, Nathalie**
**Chaussée de Stockel 67**
**B-1200 Bruxelles (BE)**
Inventeur : **Ruiz, Patricio**
**Clos du Général 1**
**B-1340 Ottignies (BE)**
Inventeur : **Delmon, Bernard**
**Avenue des Merisiers 17**
**B-1342 Ottignies-Louvain-la-Neuve (BE)**

(74) Mandataire : **De Palmenaer, Roger et al**
**Bureau Vander Haeghen S.A. Rue Colonel Bourg 108 A**
**B-1040 Bruxelles (BE)**

## (54) Système catalytique pour l'oxydation de composé organique et procédé de préparation d'un tel système.

(57) Système catalytique pour l'oxydation, l'ammoxydation et l'oxydation-déshydrogénation de composé organique, ce système comprenant une première phase constituée de $Sb_2O_4$ et une deuxième phase contenant du fer et de l'oxygène, caractérisé en ce qu'il comprend une troisième phase constituée de $MoO_3$.

EP 0 563 025 A1

La présente invention a pour objet un système catalytique pour l'oxydation de composé organique, ce système contenant au moins de l'oxygène, de l'antimoine et du fer.

On connaît par le document US-A-4 190 608 des catalyseurs pour l'oxydation sélective d'oléfines en acides et aldéhydes insaturés. Ces catalyseurs ne contiennent pas d'antimoine et ne permettent pas d'obtenir une sélectivité élevée de la réaction d'oxydation. Ainsi, dans le cas de la conversion de propylène en acroléine, la sélectivité maximale en acroléine, qui peut être obtenue par un catalyseur selon le document US-A-4 190 608 contenant du zirconium, est inférieure à environ 86%.

On connaît également par le document EP-0 102 641 un catalyseur pour la transformation en phase vapeur d'isobutylène ou de butanol tertiaire en méthacroléine.

Un catalyseur selon ce document répond à la formule générale suivante :

$$Mo_{12}W_{O\ à\ 10}Bi_{0,1\ à\ 10}Fe_{0,1\ à\ 20}$$
$$à\ (Ni\ ou\ Co)_{2\ à\ 20}\ (alcalin\ ou\ alcalino\text{-}terreux)_{0\ à\ 10}$$
$$(P, Te, Sb, Sn, Ce, Pb, Ni, Mn, Zn)_{0\ à\ 4}$$
$$(Si, Al, Zr, Ti)_{0\ à\ 30}O_x$$

Ce document décrit l'utilisation d'un catalyseur répondant à la formule suivante :

$$Mo_{12}Ni_6Bi_1Fe_3Ti_1Sb_2Sn_1Te_{0,5}$$

L'utilisation d'un tel catalyseur ne permet d'obtenir qu'une sélectivité en méthacroléine inférieure à 80%.

Un tel catalyseur est riche en fer et molybdène par rapport à sa teneur en antimoine, de sorte qu'il ne présente pas une phase constituée de microcristaux de $Sb_2O_4$.

D'autres documents enseignent des catalyseurs de formule :

$$Fe_aSb_bX_cO_x$$

pour l'oxydation de composé organique.

Des essais effectués par le demandeur ont montré que, pour l'oxydation d'isobutylène en méthacroléine, de tels catalyseurs ne permettent pas d'obtenir une sélectivité en méthacroléine supérieure à 97% tout en assurant un rendement de conversion d'isobutylène très important.

La présente invention vise à remédier à cet inconvénient et a pour objet un système catalytique comprenant une première phase constituée de $Sb_2O_4$, avantageusement de particules, de préférence de microcristaux de $Sb_2O_4$, ayant avantageusement une taille inférieure à 1000 nanomètres, de préférence à 200, voire 50 nanomètres, une deuxième phase contenant du fer et de l'oxygène et une troisième phase constituée de $MoO_3$. Un tel système catalytique convient, en particulier, pour l'oxydation sélective, l'ammoxydation et l'oxydation-déshydrogénation.

Le demandeur a observé qu'il était possible d'accroître la sélectivité de l'oxydation de composé organique en utilisant un système catalytique comprenant une première phase constituée de microcristaux de $Sb_2O_4$ et une deuxième phase contenant du fer.

Dans une forme avantageuse, la deuxième phase contient du fer sous forme d'oxyde, de préférence sous forme de $Fe_2O_3$ et, en particulier, de $FeSbO_4$.

La deuxième phase, qui contient avantageusement du fer, de l'oxygène et éventuellement de l'antimoine, peut également contenir un ou plusieurs éléments choisis parmi le tellure, le cuivre, le molybdène, le nickel et le phosphore.

Dans une forme de réalisation, les première et seconde ou la seconde et partiellement la première phase forment un ensemble de formule générale suivante :

$$Fe_aSb_bX_cO_x \qquad (1)$$

dans laquelle

a et b sont des nombres supérieurs à 0, le rapport b/a étant supérieur ou égal à 0,5, avantageusement à 1, de préférence à 1,5;

X est un élément du tableau périodique de Mendeliev autre que Fe et Sb ou un dérivé dudit élément;

c est un nombre éventuellement égal à 0, et

x est un nombre satisfaisant à la valence des éléments du système catalytique.

De préférence, la seconde et au moins partiellement la première phase forment un composé de formule générale suivante :

$$Fe_cSb_dTe_{0\ à\ 10}Cu_{0\ à\ 5}P_{0\ à\ 5}Mo_{0\ à\ 6}$$
$$Ni_{0\ à\ 10}\ (SiO_2)_{0\ à\ 120}O_x \qquad (2)$$

avec

c et d des nombres supérieurs à 5, le rapport d/c étant supérieur ou égal à 1, de préférence à 1,5.

Dans une forme de réalisation avantageuse, ce composé répond à la formule générale suivante :

$$Fe_eSb_fTe_gCu_hP_iMo_jNi_k(SiO_2)_{0\ à\ 60}O_x \qquad (3)$$

avec

e un nombre compris entre 10 et 50, de préférence égal à environ 10;

f un nombre compris entre 10 et 50, de préférence égal à environ 15;

g un nombre compris entre 2 et 8, de préférence égal à environ 5;

h un nombre compris entre 0 et 2, de préférence égal à environ un nombre compris entre 0 et 2, de préférence égal à environ 0,5;

j un nombre compris entre 0 et 3, de préférence égal à environ 1,5, et

k un nombre compris entre 0 et 5, de préférence égal à environ 2.

Le demandeur a remarqué qu'un système catalytique de formules générales 1, 2 et, en particulier 3, présentait quelques microcristaux de $Sb_2O_4$ en surface.

De préférence, la deuxième phase contient de l'oxyde de fer $Fe_2O_3$, phase $\alpha$, ladite deuxième phase formant un support sur lequel du $Sb_2O_4$ est déposé.

Dans une forme de réalisation, le système catalytique comprend un support de formule générale 1, sur lequel du $Sb_2O_4$ complémentaire est déposé. De façon avantageuse, le système catalytique présente au moins en surface des particules contenant de l'antimoine, en particulier du $Sb_2O_4$, lesdites particules ayant une taille inférieure à 1000 nanomètres, avantageusement à 200 nanomètres, de préférence comprise entre 1 et 50 nanomètres.

Dans une autre forme de réalisation, le système catalytique comprend un support à base d'oxyde de molybdène, à la surface duquel se trouve du $Sb_2O_4$.

Un autre objet de la présente invention est un procédé d'oxydation sélective d'un composé organique, dans lequel une source d'oxygène, de préférence de l'air, le composé organique à oxyder et un système catalytique suivant l'invention sont mis en contact.

La réaction d'oxydation est avantageusement effectuée en phase vapeur, et éventuellement en présence de vapeur d'eau.

Le procédé suivant l'invention permet, entre autres, une oxydation sélective de composés organiques présentant au moins une double liaison ainsi que de préférence au moins une chaîne ramifiée ou ramification. De tels composés organiques sont, par exemple, des composés hydrocarbonés ramifiés, ces composés étant de préférence des composés linéaires présentant au moins une ramification alkyle, de préférence méthyle en position 2 ou en avant-dernière position.

A titre d'exemple, le composé hydrocarboné peut répondre à la formule générale suivante :

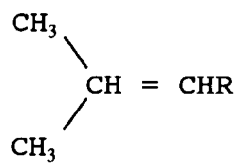

dans laquelle

R est un atome d'hydrogène ou un groupe hydrocarboné.

Des exemples préférés de composés hydrocarbonés, qui peuvent être oxydés avec une grande sélectivité en utilisant un système catalytique suivant l'invention, sont l'isobutylène, le 2-méthyl-2-butène, le 2,3-diméthyl-2-butène, le 2,4-diméthyl-2,4-hexadiène et leurs mélanges.

Les produits de réaction qu'il est ainsi possible d'obtenir avec une grande sélectivité sont la méthacroléine, la diméthylacroléine, le 2-méthyl-2-butanol, le 2,3-diméthylbutènedial et le 2,5-diméthylhexadiènedial.

De tels composés peuvent être utilisés dans la fabrication de méthylméthacrylate (précurseur du Plexiglas), de résines acryliques, de peintures, etc.

La réaction d'oxydation est avantageusement effectuée en phase gazeuse ou vapeur à une température comprise entre 200 et 600°C. Cette température est toutefois, de préférence, comprise entre 350 et 460°C, en particulier entre 400 et 460°C.

La présente invention a encore pour objet un procédé de préparation de méthacroléine par oxydation d'isobutylène en phase vapeur, dans lequel on oxyde l'isobutylène en présence d'un système catalytique suivant l'invention à une température comprise entre 200 et 600°C, de préférence comprise entre 350 et 460°C. Pour cette réaction, le rapport molaire $O_2$/isobutylène est avantageusement compris entre 2 et 4, de préférence d'environ 2,7 à 3. Lorsqu'on utilise de l'air comme source d'oxygène, le rapport $N_2$/isobutylène est avantageusement voisin de 11-12. La réaction d'oxydation est avantageusement effectuée en présence de vapeur d'eau ou d'un alcool, le rapport molaire eau-alcool/isobutylène étant, de préférence, inférieur à 0,5, en particulier inférieur à 0,2.

Une première et une deuxième phase d'un système catalytique suivant l'invention peuvent être préparées en mélangeant dans une solution, de préférence aqueuse, au moins un composé d'antimoine, de préférence

sous forme d'oxyde ou de sels, et au moins un composé de fer, de préférence sous forme d'oxyde ou de sels, en évaporant ladite solution de manière à obtenir un résidu contenant de l'antimoine et du fer et en calcinant ledit résidu.

La solution aqueuse contient, de préférence, un acide, en particulier de l'acide nitrique, de l'acide phosphorique ou un mélange de ceux-ci.

La calcination du résidu est avantageusement effectuée à une température comprise entre 200 et 700°C, mais est de préférence réalisée en au moins deux étapes, la première étant effectuée à une température inférieure à 400°C, tandis que la seconde est effectuée à une température supérieure à 400°C.

Dans une forme de réalisation d'un procédé de préparation d'une première et d'une deuxième phase d'un système catalytique suivant l'invention, on dissout dans une solution d'acide nitrique et d'eau, du fer électrolysé, et avantageusement du tellure métallique et/ou du nitrate de cuivre et/ou du nitrate de nickel et/ou de l'acide orthophosphorique, on ajoute à la solution ainsi préparée de l'oxyde d'antimoine $Sb_2O_4$ en poudre, on ajuste le pH de la solution à une valeur comprise entre 2 et 5, de préférence voisine de 3, et on évapore la solution de manière à obtenir un résidu solide que l'on calcine à une température supérieure à 200°C.

Selon une particularité de cette forme de réalisation, on ajoute à la solution contenant du fer électrolysé et éventuellement de l'oxyde d'antimoine, un dérivé de molybdène. Ce dérivé est avantageusement dissous dans une solution aqueuse que l'on ajoute à la solution contenant du fer électrolysé (c'est-à-dire dans laquelle du fer électrolysé a été introduit).

Dans le système catalytique suivant l'invention, il est avantageux d'utiliser un support sur lequel se trouve ou est déposé du $Sb_2O_4$. La présente invention a donc encore pour objet un procédé pour déposer du $Sb_2O_4$ sur un support. Dans ce procédé, on dissout de l'oxyde d'antimoine dans une solution acide, on ajoute à ladite solution un support de manière à imprégner ce dernier d'antimoine (c'est-à-dire d'antimoine sous forme de dérivé ou de sel), on évapore la solution et on calcine le support imprégné d'un composé d'antimoine de manière à obtenir un support muni de $Sb_2O_4$.

Le support utilisé dans ce procédé est avantageusement un support contenant du fer (de préférence sous forme $Fe_2O_3$ ou $FeSbO_4$), un catalyseur d'oxydation, de préférence contenant du fer ou tout autre support tel que, par exemple, un support contenant de l'oxyde de molybdène.

La solution utilisée dans ce procédé est avantageusement une solution aqueuse contenant un acide fort, en particulier de l'acide chlorhydrique.

Un autre procédé pour associer du $Sb_2O_4$ à un support ou pour déposer sur celui-ci du $Sb_2O_4$ susceptible de former au moins une phase d'un système suivant l'invention consiste à préparer une première solution contenant un alcoxyde d'antimoine, à ajouter à ladite première solution un support, de préférence sous forme dissoute dans une deuxième solution, à évaporer le mélange de la première et de la deuxième solution, de manière à obtenir un résidu, et à calciner ledit résidu à une température supérieure à 200°C.

De préférence, l'alcoxyde d'antimoine est le butoxyde d'antimoine.

L'oxyde d'antimoine de formule $Sb_2O_4$ susceptible d'être utilisé dans un procédé de préparation d'un système catalytique suivant l'invention ou d'une phase de celui-ci, est avantageusement préparé de la manière suivante.

On dissout du trichlorure d'antimoine dans une solution contenant de l'acide chlorhydrique, on ajoute ensuite à ladite solution un acide de formule :

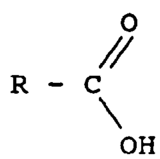

dans laquelle

R est un groupe hydrocarboné éventuellement substitué par un ou plusieurs groupes hydroxyle ou carboxyle et on évapore enfin la solution de manière à obtenir un résidu que l'on calcine.

De façon avantageuse, l'acide de formule :

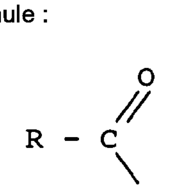

est l'acide formique, acétique, citrique, maléique, tartrique, lactique ou un mélange de ceux-ci.

D'autres particularités et détails de l'invention ressortiront de la description détaillée d'exemples de préparation et d'utilisation de systèmes catalytiques suivant l'invention.

Pour la préparation de système catalytique suivant l'invention, on a avantageusement utilisé des matières de départ choisies parmi :

- les composés de molybdène tels que l'acide molybdique, l'heptamolybdate d'ammonium et le trioxyde de molybdène;
- les composés de fer tels que le nitrate de fer, le chlorure de fer, l'oxyde de fer et le fer métallique éventuellement obtenu par électrolyse;
- les composés d'antimoine tels que le trichlorure d'antimoine, le trioxyde d'antimoine;
- les composés de tellure tels que l'acide tellurique, le chlorure de tellure et le tellure métallique;
- les composés de cuivre tels que le nitrate de cuivre et le chlorure de cuivre;
- les composés de nickel tels que le nitrate de nickel et le chlorure de nickel.

Exemple de préparation 1

On a mélangé 150 ml d'acide nitrique concentré et 200 ml d'eau. Dans le mélange ainsi formé, on a dissous successivement 4 g de fer électrolysé en poudre et 4,56 g de tellure métallique. On a ensuite ajouté 1,41 g de nitrate de cuivre, 4,6 g de nitrate de nickel et enfin 0,42 g d'acide phosphorique (pureté de 85%). On a ainsi obtenu une première solution contenant du Fe, Te, Cu, Ni et P.

On a préparé une deuxième solution en mélangeant 1,9 g d'heptamolybdate d'ammonium dans 100 ml d'eau.

Après mélange des première et deuxième solutions, on y a ajouté 15,68 g de $Sb_2O_4$ qui a été préparé de la manière décrite dans l'exemple de préparation d'oxydation d'antimoine.

Après avoir maintenu la solution sous agitation pendant 4 heures, on a ajusté le pH à 3 à l'aide de $NH_4OH$ et on a chauffé au reflux la solution pendant 2 heures et 30 minutes.

Ensuite, on a éliminé l'eau au rotavapeur sous vide et on a séché le résidu solide obtenu après élimination d'eau, à une température de 110°C. Le solide a enfin été calciné à 200°C pendant 2 heures, à 400°C pendant 2 heures et à 700°C pendant 4 heures.

On a ainsi obtenu un système catalytique de formule :

$$Fe_{10}Sb_{15}Te_5Cu_1P_{0,5}Mo_{1,5}Ni_2O_x \qquad (10)$$

Exemple de préparation 2

Après avoir préparé un système catalytique de formule :

$$Fe_{10}Sb_{15}Te_5Cu_1P_{0,5}Mo_{1,5}Ni_2O_x \qquad (10)$$

de la manière décrite dans l'exemple de préparation 1, on a imprégné ce système catalytique de la manière suivante.

On a dissous 0,03 g de $Sb_2O_4$ dans 100 ml d'eau et quelques ml d'acide chlorhydrique concentré. On a ajouté à cette solution 2 g de système catalytique de formule 10 et la solution a ensuite été agitée pendant plusieurs heures.

On a alors évaporé la solution au rotavapeur sous vide et, après séchage à 110°C du résidu solide obtenu, on a calciné le solide à 500°C pendant 4 heures. On a ainsi obtenu une poudre d'un système catalytique répondant à la formule suivante :

$$Fe_{10}Sb_{15}Te_5Cu_1P_{0,5}Mo_{1,5}Ni_2O_x + 1,5\ \%\ Sb_2O_4 \qquad (11)$$

Ce système catalytique se présentait, après tamisage, sous la forme de grains ayant une granulométrie comprise entre 0,5 et 0,8 mm, lesdits grains présentant en surface des microcristaux de $Sb_2O_4$.

Exemple de préparation 3

Dans un mélange de 150 ml d'acide nitrique concentré et de 200 ml d'eau, on a dissous 4 g de poudre de fer électrolysé et 4,56 g de tellure métallique. On a ensuite ajouté 1,41 g de nitrate de cuivre et 0,41 g d'acide phosphorique (pureté à 85%) de manière à obtenir une solution A.

Après avoir préparé une solution B en dissolvant 1,9 g d'heptamolybdate dans 100 ml d'eau, on a mélangé cette solution B à la solution A et maintenu ce mélange sous agitation pendant plusieurs heures.

On a alors ajouté 15,68 g de $Sb_2O_4$ et maintenu le mélange sous agitation pendant plusieurs heures. Après cette agitation, on a ajusté le pH du mélange à environ 3 au moyen de $NH_4OH$. Après chauffage au reflux de la solution pendant 2,5 heures, on a évaporé la solution sous vide au rotavapeur de manière à obtenir un résidu

solide que l'on a séché à 110°C pendant plusieurs heures, calciné à 200°C pendant 2 heures, à 400°C pendant 2 heures et à 700°C pendant 4 heures. On a ainsi obtenu un système catalytique de formule :

$$Fe_{10}Sb_{15}Te_5Cu_1P_{0,5}Mo_{1,5}O_x \quad (12)$$

Après tamisage de ce système catalytique pour récupérer des grains ayant une granulométrie comprise entre 0,5 et 0,8 mm, on a déposé sur ces grains 1,5% en poids de microcristaux de $Sb_2O_4$ de la manière décrite dans l'exemple 2.

On a ainsi obtenu un système catalytique de formule :

$$Fe_{10}Sb_{15}Te_5Cu_1P_{0,5}Mo_{1,5}O_x + 1,5\ \%\ Sb_2O_4 \quad (13)$$

Exemple de préparation 4

On a préparé un support d'oxyde de molybdène portant des particules de $Sb_2O_4$ de la manière suivante.

On a calciné 5 g d'heptamolybdate d'ammonium à 500°C pendant 6 heures de manière à obtenir un support d'oxyde de molybdène.

On a dissous 0,03 g de $Sb_2O_4$ dans 100 ml d'eau additionnée de quelques ml d'acide chlorhydrique concentré et on a ajouté à cette solution 2 g de support d'oxyde de molybdène. Après agitation du mélange pendant plusieurs heures, on a évaporé l'eau au rotavapeur sous vide, séché le résidu solide à 110°C pendant quelques heures et calciné le résidu à 500°C pendant 4 heures.

On a ainsi obtenu un support de formule :

$$MoO_3 + 1,5\%\ Sb_2O_4 \quad (18)$$

que l'on a tamisé pour récupérer les grains dont la granulométrie est comprise entre 0,5 et 0,8 mm.

Exemple de préparation 5

Après avoir préparé un système catalytique de formule 12 de la manière décrite à l'exemple 3, on a déposé sur celui-ci de l'antimoine à partir d'un alcoxyde d'antimoine, à savoir le n-butoxyde d'antimoine.

Pour effectuer cette opération, on a ajouté goutte à goutte 2 ml de n-butoxyde d'antimoine à 400 ml d'eau. Après avoir agité cette solution à température ambiante pendant 22 heures, on a éliminé au rotavapeur sous vide 50% d'eau de ladite solution.

On a mélangé 2 g du système catalytique de formule 12 dans 1 litre d'eau et on y a ajouté la solution d'alcoxyde goutte à goutte. Après mélange de la solution à température ambiante pendant 1 heure, on a évaporé le solvant à 60°C tout en continuant l'agitation pendant 24 heures. Le restant d'eau est éliminé par évaporation à 120°C. On a ainsi récupéré un résidu solide que l'on a séché à 110°C et calciné en trois étapes, à savoir une première étape à 200°C (2 heures), une deuxième étape à 400°C (2 heures) et enfin une dernière étape à 700°C (4 heures).

On a ainsi obtenu un système catalytique de formule :

$$Fe_{10}Sb_{15}Te_5Cu_1P_{0,5}Mo_{1,5}O_x + Sb_2O_4 \quad (15)$$

Exemple de préparation 6

Dans un mélange de 150 ml d'acide nitrique concentré et 200 ml d'eau, on a dissous 4 g de fer électrolysé en poudre et 4,56 g de tellure métallique. On a ensuite ajouté 1,41 g de nitrate de cuivre et 0,42 g d'acide phosphorique (pureté de 85%) de manière à obtenir une première solution contenant du Fe, Ti, Cu et P.

On a préparé une deuxième solution en mélangeant 1,9 g d'heptamolybdate d'ammonium dans 129,14 g d'une solution aqueuse contenant 20% en poids de silice.

Après mélange des première et deuxième solutions, on a ajouté 15,68 g de $Sb_2O_4$ en poudre et on a ajusté le pH à 3 au moyen de $NH_4OH$. La solution ainsi obtenue a été chauffée au reflux pendant 2,5 heures. L'eau est alors évaporée au rotavapeur sous vide. On a ensuite séché le résidu solide à 110°C pendant plusieurs heures avant de le calciner à 200°C pendant 2 heures, à 400°C pendant 2 heures et enfin à 700°C 4 heures.
On a ainsi obtenu un système catalytique de formule :

$$Fe_{10}Sb_{15}Te_5Cu_1P_{0,5}Mo_{1,5}O_{61,8}(SiO_2)_{60} \quad (16)$$

Après tamisage dudit système catalytique pour récupérer la fraction granulométrique 0,5-0,8 mm, on a ajouté 2 g dudit système catalytique à une solution aqueuse contenant quelques ml d'acide chlorhydrique concentré et 0,03 g de $Sb_2O_4$. Après mélange de ladite solution pendant plusieurs heures, on a évaporé l'eau sous vide et séché le résidu solide à 110°C pendant plusieurs heures. On a ensuite calciné ledit résidu solide à 500°C pendant 4 heures de manière à obtenir un système catalytique de formule :

$$Fe_{10}Sb_{15}Te_5Cu_1P_{0,5}Mo_{1,5}O_{61.8}(SiO_2)_{60} + 1,5\%\ Sb_2O_4 \quad (17)$$

Exemples d'utilisation

Dans ces exemples, on a placé 300 mg d'un système catalytique (granulométrie 0,5-0,8 mm) dans un réacteur tubulaire en forme de U et on a fait passer sur ce catalyseur un mélange gazeux d'isobutylène, d'oxygène, d'azote et éventuellement de vapeur d'eau à une température voisine de 460°C. Le débit total du mélange gazeux était de 37,7 ml/minute.

On a déterminé pour différents systèmes catalytiques, la conversion de l'isobutylène ( oxydation totale, méthacroléine, etc. ) C Iso, le rendement en méthacroléine "Métha" et la sélectivité en méthacroléine "S Métha ".

Ces différents paramètres peuvent être déterminés par les formules suivantes :

$$C_{Iso} \text{ en } \% = \frac{\text{nombre de moles d'isobutylène consommées}}{\text{nombre initial de moles d'isobutylène}} \times 100$$

$$\eta_{Métha} \text{ en } \% = \frac{\text{nombre de moles de méthacroléine produites}}{\text{nombre initial de moles d'isobutylène}} \times 100$$

$$S_{Métha} \text{ en } \% = \frac{\text{nombre de moles de méthacroléine produites}}{\text{nombre de moles d'isobutylène consommées}} \times 100$$

Le tableau 1 suivant donne, pour différents catalyseurs, le rapport molaire isobutylène, oxygène, azote, vapeur du mélange gazeux, la conversion, le rendement en méthacroléine et la sélectivité en méthacroléine.

| Système catalytique de formule | Isobutylène/$O_2$/$N_2$/$H_2O$ | $C_{Iso}$ | $\eta_{Métha}$ | $S_{Métha}$ |
|---|---|---|---|---|
| $Fe_{10}Sb_{15}Te_5Cu_1P_{0,5}Mo_{1,5}Ni_2O_x$ (10) | 1/2,7/11,5/0<br>1/2,7/11,5/0,03 | 67<br>92,3 | 58,2<br>87,6 | 86,8<br>94,4 |
| $Fe_{10}Sb_{15}Te_5Cu_1P_{0,5}Mo_{1,5}Ni_2O_x$ + 1,5% $Sb_2O_4$ (11) | 1/2,7/11,5/0 | 65,2 | 58,2 | 89,4 |
| $Fe_{10}Sb_{15}Te_5Cu_1P_{0,5}Mo_{1,5}O_x$ + 1,5% $Sb_2O_4$ (13) | 1/2,7/11,5/0 | 81,6 | 79 | 96,8 |
| (18 = $MoO_3$ + 1,5% $Sb_2O_4$) (13) + (18)<br>[mélange 50% (13) - 50% (18)] | 1/2,7/11,5/0 | 89,8 | 88,4 | 98,4 |
| $Fe_{10}Sb_{15}Te_5Cu_1P_{0,5}Mo_{1,5}O_x$ + $Sb_2O_4$ (15) | 1/2,7/11,5/0 | 81,4 | 77,3 | 94,9 |
| $Fe_{10}Sb_{15}Te_5Cu_1P_{0,5}Mo_{1,5}O_{61,8}(SiO_2)_{60}$ + 1,5% $Sb_2O_4$ (17) | 1/2,7/11,5/0 | 87,5 | 77,9 | 89 |

Ce tableau montre que le système catalytique constitué de particules à base de fer portant des microcristaux de $Sb_2O_4$ et de particules de $MoO_3$ portant des microparticules de $Sb_2O_4$, (les microparticules n'enveloppant pas complètement les particules), permet d'obtenir une sélectivité en méthacroléine de plus de 98% tout en assurant un excellent rendement de conversion d'isobutylène. Ceci permet donc de réduire au maximum les étapes de purification de la méthacroléine et les quantités de réactifs de départ.

Pour une installation existante, cela signifie que, grâce à un accroissement de la vitesse de réaction, il est possible d'accroître la capacité de production d'une installation existante sans requérir d'investissement. De plus, le système catalytique, suivant l'invention, présentait une meilleure stabilité ou activité dans le temps, c'est-à-dire une durée de vie plus importante.

Enfin, le système, suivant l'invention, permet d'éviter d'utiliser des quantités de catalyseurs à base de Fe, Sb, $O_2$ coûteux.

Exemple de préparation d'oxyde d'antimoine convenant pour l'imprégnation de support

On a dissous 40 g de trichlorure d'antimoine dans 100 ml d'eau et quelques ml d'acide chlorhydrique concentré. On a ensuite ajouté à la solution ainsi formée 100 ml d'acide nitrique concentré. Après mélange de la solution formée, on a ajouté 36,84 g d'acide citrique et on a agité la solution pendant une nuit. On a évaporé l'eau au rotavapeur sous vide et on a obtenu une meringue brunâtre que l'on a séchée à 110°C sous vide pendant plusieurs heures. Lors de cette opération, la meringue a continué de gonfler. Ensuite, on a séché la meringue à 500°C pendant 20 heures. Après broyage, on a ainsi obtenu une poudre de $Sb_2O_4$.

Il est à noter que le $Sb_2O_4$ peut se présenter sous la forme d'une couche couvrant au moins partiellement du $Sb_2O_3$, par exemple des particules de $Sb_2O_3$ d'une granulométrie inférieure à 1000 nanomètres.

Enfin, il va de soi que le support à base de Fe et $O_2$ et/ou le support à base de $MoO_3$ peuvent, dans des formes de réalisation, être modifiés par d'autres éléments ou métaux, en particulier déposés en surface (par exemple Cu, Pt, Au, Ag, ...).

## Revendications

**1.-** Système catalytique pour l'oxydation, l'ammoxydation et l'oxydation-déshydrogénation de composé organique, ce système comprenant une première phase constituée de $Sb_2O_4$ et une deuxième phase contenant du fer et de l'oxygène, caractérisé en ce qu'il comprend une troisième phase constituée de $MoO_3$.

**2.-** Système catalytique suivant la revendication 1, caractérisé en ce que la deuxième phase contient du fer sous forme de $FeSbO_4$ ou de $Fe_2O_3$.

**3.-** Système catalytique suivant la revendication 1 ou 2, caractérisé en ce que la deuxième phase et au moins en partie la première phase forment un composé de formule générale suivante :

$$Fe_cSb_dTe_{0\text{ à }10}Cu_{0\text{ à }5}P_{0\text{ à }5}Mo_{0\text{ à }6}$$
$$Ni_{0\text{ à }10}(SiO_2)_{0\text{ à }120}O_x \qquad (2)$$

avec

c et d des nombres supérieurs à 5, le rapport d/c étant supérieur ou égal à 1.

**4.-** Système catalytique suivant l'une des revendications 1 à 3, caractérisé en ce que la deuxième phase contient du $Fe_2O_3$, phase $\alpha$, et forme un support sur lequel du $Sb_2O_4$ est déposé ou se trouve du $Sb_2O_4$.

**5.-** Système suivant l'une des revendications 1 à 4, caractérisé en ce que l'oxyde de molybdène forme un support sur lequel se trouve du $Sb_2O_4$.

**6.-** Système suivant la revendication 5, caractérisé en ce qu'il comprend, d'une part, des particules contenant du fer et de l'oxygène à la surface desquelles se trouvent des particules de $Sb_2O_4$ et, d'autre part, des particules de $MoO_3$ à la surface desquelles se trouvent des particules de $Sb_2O_4$.

**7.-** Utilisation d'un système suivant l'une quelconque des revendications 1 à 6 pour l'oxydation, l'ammoxydation et l'oxydation-déshydrogénation de composé organique.

**8.-** Procédé de préparation de méthacroléine par oxydation d'isobutylène en phase vapeur, dans lequel on effectue l'oxydation en présence d'un système catalytique suivant l'une quelconque des revendications 1 à 6 en phase gazeuse à une température comprise entre 200 et 600°C.

**9.-** Procédé pour déposer du $Sb_2O_4$ sur un support susceptible de former au moins une phase d'un système suivant l'une quelconque des revendications 1 à 6, dans lequel on dissout de l'oxyde d'antimoine dans une solution acide, on ajoute à ladite solution un support de manière à imprégner ce dernier d'antimoine, on évapore la solution et on calcine le support imprégné d'un composé d'antimoine de manière à obtenir un support muni de $Sb_2O_4$.

**10.-** Procédé suivant la revendication 9, caractérisé en ce que le support est un support contenant du fer,

un catalyseur d'oxydation contenant du fer ou un support contenant un oxyde de molybdène.

**11.-** Procédé pour associer du $Sb_2O_4$ à un support susceptible de former au moins une phase d'un système suivant l'une quelconque des revendications 1 à 6, dans lequel on prépare une première solution contenant un alcoxyde d'antimoine, de préférence du butoxyde d'antimoine, on ajoute à ladite première solution un support, de préférence sous forme dissoute, dans une deuxième solution, on évapore la première et éventuellement la deuxième solution de manière à obtenir un résidu et on calcine ledit résidu à une température supérieure à 200°C.

**12.-** Procédé de préparation d'oxyde d'antimoine de formule :

$$Sb_2O_4$$

susceptible de former une phase d'un système suivant l'une quelconque des revendications 1 à 6, dans lequel on dissout du trichlorure d'antimoine dans une solution contenant de l'acide chlorhydrique, on ajoute à ladite solution un acide de formule :

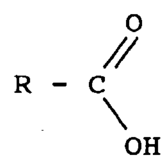

dans laquelle

R est un groupe hydrocarboné éventuellement substitué par un ou plusieurs groupes hydroxyle ou carboxyle et on évapore enfin la solution de manière à obtenir un résidu que l'on calcine.

**13.-** Procédé suivant la revendication 12, caractérisé en ce que l'acide de formule :

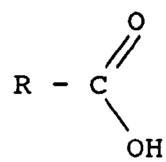

est l'acide formique, acétique, citrique, maléique, tartrique, lactique ou un mélange de ceux-ci.

**14.-** Utilisation de particules de $MoO_3$ recouvertes de $Sb_2O_4$ pour améliorer la sélectivité de catalyseur d'oxydation.

Office européen des brevets

# RAPPORT DE RECHERCHE EUROPEENNE

Numero de la demande

EP 93 87 0054
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 109 259 (NITTO)<br>* le document en entier *<br>--- | 1-14 | B01J23/84 |
| A | US-A-2 465 313 (MOSESMAN)<br>* le document en entier *<br>--- | 4 | |
| A | EP-A-0 102 641 (NIPPON SHOKUBAI)<br>* le document en entier *<br>--- | 7,8 | |
| A | EP-A-0 323 129 (NITTO)<br>* le document en entier *<br>--- | 3,9,10 | |
| A | US-A-4 040 983 (INNES ET AL.)<br>* le document en entier *<br>--- | 12,13 | |
| A | EP-A-0 088 328 (BASF)<br>* le document en entier *<br>--- | 12,13 | |
| A | EP-A-0 180 997 (MITSUBISHI RAYON)<br>* le document en entier *<br>--- | 1 | |
| A | EP-A-0 337 028 (STANDARD OIL)<br>* le document en entier *<br>--- | 1-14 | **DOMAINES TECHNIQUES RECHERCHES (Int. Cl.5)** |
| A | US-A-3 758 609 (CICHOWSKI)<br>* le document en entier *<br>--- | 12,13 | B01J |
| A | EP-A-0 153 077 (NITTO)<br>* le document en entier *<br>--- | 1-14 | |
| A | EP-A-0 036 464 (STANDARD OIL)<br>* le document en entier *<br>--- | 1-14 | |
| A | FR-A-2 279 706 (STANDARD OIL)<br>* le document en entier *<br>--- | 1-14 | |
| A | EP-A-0 319 192 (NITTO)<br>* le document en entier *<br>---<br>-/-- | 1-14 | |

Le présent rapport a été établi pour toutes les revendications

| Lieu de la recherche | Date d'achèvement de la recherche | Examinateur |
|---|---|---|
| LA HAYE | 25 JUIN 1993 | DE LA MORINERIE |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 93 87 0054
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 111, no. 25, 18 Décembre 1989, Columbus, Ohio, US; abstract no. 231723q, WENG ET AL. 'COOPERATION BETWEEN OXIDE PHASES IN SELECTIVE OXIDATION OF ISOBUTENE TO METHACROLEIN.' page 696 ;colonne 1 ; & PROC.-INT.CONGR.CATAL.,9TH 1988,4,1609-16.ED. BY PHILLIPS,M.J.;TERNAN,M. CHEM.INST.CAN.:OTTAWA,ONT. * abrégé * | 1-14 | |
| | ----- | | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 25 JUIN 1993 | DE LA MORINERIE |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)